# EUROPEAN PATENT APPLICATION

(11) **EP 3 270 331 A1**
(43) Date of publication of application: **17.01.2018**
(21) Application number: 16179657.8
(22) Date of filing: 15.07.2016
(51) Int. Cl.: G06Q 10/00, G06Q 50/24

(54) **A MEDICAL CONSULTATION SUPPORT TOOL**

(71) Applicant: Little Brain NV, 2020 Antwerpen (BE)
(72) Inventor: Van de Steen, Piet, 2020 Antwerpen (BE); Van De Putte, Tom, 9900 Eeklo (BE)
(74) Representative: Plas, Axel Ivo Michel

(57) **Abstract**

A medical consultation support tool (100) comprises:
- a database (101) of arguments and diagnoses wherein incidence values (I1, I2, I3, I4), sensitivity values (SA1, SA2), and aspecificity values (AA1, AA2 are maintained;
- an electronic patient portal (102) adapted to obtain a pre-consultation complaint description (152) from a patient;
- a differential diagnosis module (103) adapted to generate a differential diagnosis (156) from the pre-consultation complaint description (152);
- an electronic clinician portal (104), adapted to obtain post-consultation diagnosis and confidence information (158) from a clinician; and
- a processor (105) configured to automatically adapt incidence values (I1, I2, I3, I4), sensitivity values (SA1, SA2) and aspecificity values (AA1, AA2) with respective amounts calculated from the post-consultation diagnosis and confidence information (158) obtained from the clinician.

## Description

### Field of the Invention

The present invention generally relates to technical support in medical consultation and diagnosis.

### Background of the Invention

Scheduling medical consultation, i.e. planning appointments between patient and clinician, and collecting personal health information from the patient are time consuming, repetitive actions that often cause frustration with the patient and clinician. The patient gets frustrated as he spends a lot of time in the clinician's waiting room - 40 minutes average waiting time according to certain studies, even if an appointment is scheduled - and each consultation starts with the collection of repetitive information that could form part of the patient's personal health record (e.g. the family history, life style, etc.). The clinician gets frustrated because the patient is often not capable of accurately explaining the real problem and/or describing his symptoms, often has a list of independent problems or complaints saved up, e.g. headache, knee injury, pain in the back, ..., and often mentions the main problem or complaint only near the end of the consultation (door-knob syndrome).

Tools exist that rely on information received from the patient and a database that correlates symptoms with diagnoses to generate a differential diagnosis, i.e. a list of 10 to 20 potential diagnoses, that is presented to the clinician. Examples of such tools are described in United States patent application US2015120319 entitled "System and Method for Improving Medical Diagnoses and Treatments", United States patent application US2009062623 entitled "Identifying Possible Medical Conditions of a Patient", United States patent application US2012102405 entitled "System and Method for Matching Person-Specific Data with Evidence Resulting in Recommended Actions", European patent application EP1378853 entitled "Digital Medical Assistance System", United States patent application US7149756 entitled "System and Method for Determining the Probable Existence of Disease" and United States Patent Application US6149585 entitled "Diagnostic Enhancement Method and Apparatus".

The existing systems however provide no feedback on the usefulness of the differential diagnosis. The existing systems also do not improve over time by taking benefit of knowledge on the usefulness of the differential diagnosis.

The best existing solution seems to be described in Korean patent application KR100737382 entitled "Operation Method for Medical Diagnosis Data". KR100737382 describes a medical diagnosis tool that interrogates a patient, compares the received information with a symptom list in a database and outputs a diagnosis. The system further obtains diagnosis result information from a doctor and compares this with its own diagnosis to further improve its accuracy.

It is an objective of the present invention to disclose a medical consultation support tool with enhanced accuracy and reliability.

### Summary of the Invention

According to the present invention, the above identified objective is realized by a medical consultation support tool as defined by claim 1, the medical consultation support tool comprising:
- a database of arguments and diagnoses wherein an incidence value is maintained for each diagnosis, i.e. a value indicating the occurrence probability of the disease corresponding to said diagnosis, and wherein an argument and diagnosis are relationally coupled through a sensitivity value, i.e. a value indicating how often the symptom is present in case a patient has the disease corresponding to the diagnosis, and through an aspecificity value, i.e. a value indicating how often the symptom is present in case a patient does not have the disease corresponding to the diagnosis;
- an electronic patient portal adapted to obtain a pre-consultation complaint description from a patient;
- a differential diagnosis module adapted to generate a differential diagnosis through matching arguments identified in the pre-consultation complaint description with arguments present in the database and determining potential diagnoses from incidence values, sensitivity values and aspecificity values, wherein the differential diagnosis comprises a list of potential diagnoses and respective probabilities of the potential diagnoses;
- an electronic clinician portal adapted to obtain post-consultation diagnosis information and confidence information from a clinician; and
- a processor configured to automatically adapt incidence values in the database for diagnoses in the post-consultation diagnosis information, sensitivity values and aspecificity values in the database for arguments identified in the pre-consultation complaint description with respective amounts calculated from the post-consultation diagnosis information and the confidence information obtained from the clinician.

Thus, the tool according to the present invention relies on a database that correlates arguments with diagnoses. The arguments can be symptoms, like for instance headache, fever, low blood pressure, etc., or patient data that may have an effect on the disease like gender, age, risk factors, medical history, etc. The strength of the correlation between an argument and diagnosis is expressed by two parameters: the sensitivity value (if a person has a disease, how often will the argument be present) and the aspecificity value (if a person does not have the disease, how often will the argument be present). The tool further has an electronic patient portal to collect patient information. The patient's disease-specific symptoms are collected into a Personal Health Record. The Personal Health Record further comprises information that is not disease-specific like for instance the patient's age, gender, weight, blood group, medication, allergies, life style, etc. A diagnostic module that forms part of the tool according to the present invention thereupon uses a Bayesian statistic algorithm to generate a differential diagnosis that is presented to the clinician, i.e. a list of for instance 10 to 20 potential diagnoses.

According to an advantageous aspect of the present invention, the incidence values, sensitivity values and aspecificity values in the database are made adaptive. Thereto, a clinician portal is provided that interrogates the clinician with respect to his diagnosis and confidence in his diagnosis, e.g. tentative, confirmed through tests (e.g. blood sample analysis, medical image), confirmed through treatment, etc. The incidence value associated with the clinicians diagnosis is increased. Further, the sensitivity values and aspecificity values correlating arguments identified in the patient's initial complaint description and potential diagnoses that form part of the differential diagnosis are automatically updated in view of the clinicians diagnosis. The increase of the sensitivity values and aspecificity values depends on the clinicians confidence in his diagnosis. As a result of the automatic adaptation of the sensitivity and aspecificity values in the database, the tool according to the invention has self-learning capacities and future differential diagnoses will become more accurate: at present, clinician's diagnoses are 80 % à 85 % accurate. The differential diagnoses will achieve above 90 % accuracy through the adaptive incidence values, sensitivities and aspecificities underlying the present invention.

According to an optional aspect of the medical consultation support tool according to the present invention, defined by claim 2:
- the differential diagnosis module and the electronic patient portal are further adapted to present to the patient one or more additional arguments, to obtain confirmation or denial for the additional arguments from the patient, and to generate an update of the differential diagnosis through matching the arguments identified in the pre-consultation complaint description and the additional arguments confirmed or denied by the patient with arguments present in the database; and
- the processor is further configured to automatically adapt sensitivity values and aspecificity values in the database for the additional arguments with respective amounts calculated from the post-consultation diagnosis information and the confidence information obtained from the clinician.

Indeed, embodiments of the tool according to the present invention may select additional arguments from the database that are strongly correlated with potential diagnoses and interrogate the patient with respect to those additional arguments via anamnestic questions, e.g. "do you also have ...?". The tool may for instance implement three rounds wherein respectively five, three and two additional arguments are presented to the patient and the patient is requested to confirm or deny presence of those additional arguments. The additional arguments presented to the patient through these automatically generated anamnestic questions are selected as the arguments in the database that have the highest sensitivity values or the highest aspecificity values in correlation with the potential diagnoses that form part of the differential diagnose. The patient's feedback to arguments having the highest sensitivities in relation to a potential diagnose help in finding confirmation for such a potential diagnose. The patient's feedback to arguments having the highest aspecificities in relation to a potential diagnose help in ruling out such potential diagnose. Consequently, the tool according to the present invention shall generate an updated differential diagnosis and shall also adapt the sensitivity values and aspecificity values correlating these additional arguments with the potential diagnoses in the differential diagnosis with respective amounts calculated from the clinician's diagnosis information obtained after consultation and the clinician's confidence in his diagnosis. The ability to automatically generate anamnestic questions shall improve the accuracy and reliability of the differential diagnose substantially, and further contributes significantly to the gain in time for diagnosing a patient.

Following a further optional aspect of the medical consultation support tool according to the present invention, defined by claim 3:
- the electronic clinician portal is further adapted to enable the clinician to add one or more additional arguments;
- the differential diagnosis module is further adapted to re-generate a differential diagnosis through matching the arguments identified in the pre-consultation complaint description and the additional arguments with arguments present in the database and determining potential diagnoses from incidence values, sensitivity values and aspecificity values; and
- the processor is further configured to automatically adapt sensitivity values and aspecificity values in the database for the additional arguments added with respective amounts calculated from the post-consultation diagnosis information and the confidence information obtained from the clinician.

Hence, embodiments of the present invention allow the clinician to enter additional arguments in the database, for instance symptoms identified during consultation such as a high blood pressure, a high cholesterol value, etc. Based on these additional arguments entered by the clinician and possible additional arguments obtained through anamnestic interrogation of the patient, the tool may generate a new differential diagnosis, i.e. a new list of potential diagnoses. Consequently, the sensitivity values and aspecificity values will not only be adapted for arguments identified in the patient's initial complaint description but will also be generated and adapted for the additional arguments entered by the clinician or identified through the automated anamnestic patient interrogation and potential diagnoses that form part of the new differential diagnosis. The amounts of change of the sensitivity and aspecificity values will be calculated from the clinician's final diagnosis information and the confidence in his diagnosis as expressed by the clinician via the clinician portal.

In embodiments of the medical consultation support tool according to the present invention, defined by claim 4, the processor is configured to increase a sensitivity value for arguments identified in the pre-consultation complaint description or additional arguments confirmed by the patient or added by the clinician and a diagnosis corresponding with the diagnosis information of the clinician, the increase amount of the sensitivity value being determined by the confidence information of the clinician.

Indeed, the correlation between arguments, identified or confirmed by the patient in the pre-consultation process or identified by the clinician during consultation, and the final diagnosis of the clinician is preferably increased in the database. This way, the database of arguments and diagnoses learns from clinicians and becomes more accurate over time. The increase of the correlation, i.e. the increase of the sensitivity value between such arguments and the final diagnosis, is preferably dependent upon the confidence that the clinician has in his final diagnosis. In case of low confidence, the correlation between arguments and final diagnosis can be tightened slightly, whereas in case of strong confidence, the correlation between arguments and final diagnosis can be tightened substantially. The increase value for the sensitivities in other words preferably is a growing function of the confidence value, e.g. a linear proportional function thereof.

In embodiments of the medical consultation support tool according to the present invention, defined by claim 5, the processor is configured to increase an aspecificity value for arguments identified in the pre-consultation complaint description or additional arguments confirmed by the patient or added by the clinician and a potential diagnosis that forms part of the differential diagnosis but differs from the diagnosis information of the clinician, the increase amount of the aspecificity value being determined by the confidence information of the clinician.

Indeed, the aspecificity coupling between arguments, either identified or confirmed by the patient in the pre-consultation process or identified by the clinician during consultation, and a wrong diagnosis, i.e. a diagnosis that is not elected by the clinician as final diagnosis, is preferably increased. This way, the database again takes benefit of the clinician's final diagnosis to increase its accuracy. By increasing the aspecificities, the chance that the diagnosis becomes listed as a potential diagnosis in a future differential diagnosis generated for a patient with equal or similar arguments, is reduced. Just like with sensitivities for the final diagnosis, the increase in aspecificities for wrong diagnoses is preferably a growing function of the clinician's confidence in his final diagnosis. Again, a linear proportional function, or alternate growing function can be considered to calculate the aspecificity increase from the confidence value.

In embodiments of the medical consultation support tool according to the present invention, defined by claim 6, the processor is configured to increase an incidence value for a diagnosis that corresponds with the diagnosis information of the clinician, the increase amount of the incidence value being determined by the confidence information of the clinician.

Indeed, in addition to sensitivity values and aspecificity values correlating arguments and diagnoses in the database, also the incidence values of diagnoses are automatically adapted thereby enhancing the self-learning capacity and accuracy of the medical consultation support tool. More precisely, the incidence value of the final diagnosis of the clinician shall be increased, preferably with an increase value that depends on the confidence value. The increase value shall be a rising function of the clinician's confidence value, for instance a linear function of the confidence value. It is noticed that other parameters, like seasonal changes, may influence the increase values of incidence, sensitivity and aspecificity parameters.

Further optionally, in embodiments of the medical consultation support tool according to the present invention, as defined by claim 7:
- the processor is configured to add a new diagnosis to the database in case a diagnosis corresponding with the diagnosis information of the clinician is not present in the database; and
- the processor is further configured to create sensitivity values and aspecificity values in the database for arguments identified in the pre-consultation complaint description or additional arguments and the new diagnosis.

Thus, when a clinician makes a final diagnosis that differs from all potential diagnoses in the differential diagnosis and that differs from any diagnosis in the database of diagnoses and arguments, this final diagnosis is entered as a new diagnosis in the database. Moreover, couplings are established between such new diagnosis and the arguments identified in the information received from the patient during the pre-consultation process as well as the arguments identified by the clinician during consultation. The couplings take the form of sensitivity values and aspecificity values.

In accordance with another optional aspect of the present invention, defined by claim 8, the medical consultation support tool comprises:
- a natural language processor coupled to the patient portal and adapted to process free text or speech portions of the pre-consultation complaint description to identify one or more arguments therein.

Indeed, advanced embodiments of the medical consultation support tool have an embedded natural language processor (NLP) that is programmed to identify in possible free text portions or speech portions of the patient's initial complaint description symptoms or arguments contained in the symptom-diagnose database. Such arguments identified through NLP and their eventual quantification which may also be obtained through NLP shall also be used in the generation of the differential diagnosis.

In line with a further optional aspect of the medical consultation support tool according to the present invention, as defined by claim 9, the natural language processor is further adapted to quantify the arguments identified from the pre-consultation complaint description.

The natural language processor in other words preferably is not only configured to recognize words, terms or phrases that are indicative for the presence of certain arguments, but also recognizes words, terms, figures or phrases that quantify the argument. A headache may for instance be episodic, daily, chronic, ... it may be barely noticeable, mild, lightly pinching, tolerable, strong, deep, piercing, intense, horrible, excruciating, unbearable, unspeakable, unimaginable, etc. In situations where no terms or words are found that enable to quantify an argument, the patient portal may be used to interrogate the patient and obtain a quantification of an argument like a pain scale, the measured fever temperature, etc. Similarly, embodiments of the medical diagnosis support tool according to the present invention could be enhanced with an image processor that processes images delivered by the patient, for instance in case of dermatologic diseases, to identify and/or quantify certain symptoms.

According to an advantageous aspect defined in claim 10, the medical consultation support tool according to the present invention further comprises:
- an electronic patient portal for post-consultation interrogation of the patient, adapted to collect treatment effectiveness information and/or medication effectiveness information from the patient.

Thus, a patient portal may be provided to collect information from the patient after consultation, preferably even after treatment, on the effect of the treatment and eventual medication prescribed by the clinician.

As further defined by claim 11, embodiments of the medical consultation support tool according to the present invention may comprise:
- a prognostic module coupled to the electronic patient portal for post-consultation interrogation and adapted to calculate an estimated intention time for a new patient from treatment effectiveness information obtained from earlier patients with same diagnosis and prescribed medication as the new patient.

Hence, the post-consultation patient feedback obtained through the above described patient portal can be used to generate prognoses for other patients with same or similar diagnosis. Prognostic models for instance can estimate the time to recover given a diagnosis, gender, age, prescribed medication, etc., by averaging the recovery times reported by earlier patients with same diagnosis, prescribed medication, gender and age within a given range.

Advantageous embodiments of the medical consultation support tool according to the current invention, defined by claim 12, further comprise:
- a reporting module coupled to the electronic patient portal for post-consultation interrogation and adapted to generate statistical reports on side effects of medication from the medication effectiveness information obtained from patients.

Indeed, patient feedback collected after treatment and preferably made anonymous, can be used to generate statistical reports on the effectiveness of medication and/or the side effects of medication that are useful for instance for the pharmaceutical industry. Such information is at present obtained through sampling a relatively small amount of patients, but may be generated more accurately and more reliably on larger scale and in real life setting through the embodiments of the tool according to the present invention that are equipped with the patent portal to collect post-treatment information and a module that statistically processes this information.

Advantageous embodiments of the medical consultation support tool according to the present invention, as defined by claim 13, further comprise:
- an electronic appointment scheduler enabling the patient to schedule an appointment with a clinician, to automatically receive real-time updates on the appointment within a certain time interval preceding the appointment, and to automatically receive reschedule suggestions upon appointment cancellations by other patients.

Thus, the tool not only collects information from the patient prior to consultation, but preferably also serves as an advanced appointment scheduler that contains advanced features like the ability to specify the type of appointment, e.g. a new complaint, vaccination, delivery of urine sample, prescription for medication like anticonception, ..., enabling to automatically allocate an appropriate timeslot of appropriate time length. In an advantageous embodiment of the invention, the patient receives real-time updates, e.g. delays in the clinician's scheme, enabling the patient on the day of consultation to arrive later or earlier with the clinician, and schedule other activities like shopping, administration, etc. in between. Annulations from other patients that result in free timeslots are pushed automatically to patients that have scheduled a consultation shortly before or after the annulated appointment to give such patients the ability to shift their appointment to the free timeslot if this fits better with their agenda.

According to yet another optional aspect, defined by claim 14, embodiments of the medical consultation support tool according to the present invention further comprise:
- an electronic patient portal for post-consultation interrogation of the patient, adapted to collect a reformulated complaint description from the patient; and
- a processor coupled to the electronic patient portal for post-consultation interrogation and adapted to enrich the database of arguments and diagnoses with alternate argument descriptions extracted from a comparison between the pre-consultation complaint description and the reformulated complaint description.

Post-consultation interrogation of the patient, i.e. interrogation of the patient at a point in time when the patient is more literate on his/her medical problem as a result of the consultation and eventual treatment, enables to enrich the database of arguments and diagnoses with terminology that is used by patients who are illiterate on their medical problem, e.g. certain dialects or expressions. Thereto, the patient's post-consultation description of his medical problem must be compared with the pre-consultation description of his problem. The terminology used in the pre-consultation description may be absent in the database of arguments and diagnoses and can be interpreted through comparison with the post-consultation description. Dialect synonyms for certain arguments may be identified and can be added to the database to enrich the database with terminology that may be used by future patients. The database this way will become more complete and future differential prognoses will become more accurate since argument extraction from future patient's pre-consultation descriptions will be more complete.

In addition to a tool for medical consultation support as defined by claim 1, the present invention also concerns a corresponding computer-implemented method for medical consultation support as defined by claim 15, the method comprising the steps of:
- maintaining a database of arguments and diagnoses wherein an incidence value is maintained for each diagnosis, i.e. a value indicating the occurrence probability of the disease corresponding to the diagnosis, and wherein an argument and diagnosis are relationally coupled through a sensitivity value, i.e. a value indicating how often the argument is present in case a patient has the disease corresponding to the diagnosis, and an aspecificity value, i.e. a value indicating how often the argument is present in case a patient does not have the disease corresponding to the diagnosis;
- obtaining via an electronic patient portal a pre-consultation complaint description from a patient;
- generating a differential diagnosis through matching arguments identified in the pre-consultation complaint description with arguments present in the database and determining potential diagnoses from incidence values, sensitivity values and aspecificity values, wherein the differential diagnosis comprises a list of potential diagnoses and respective probabilities of the potential diagnoses;
- obtaining post-consultation diagnosis information and confidence information from a clinician via an electronic clinician portal; and
- automatically adapting incidence values in the database for diagnoses in the post-consultation diagnosis information, and sensitivity values and aspecificity values in the database for arguments identified in the pre-consultation complaint description with respective amounts calculated from the post-consultation diagnosis information and the confidence information obtained from the clinician.

### Brief Description of the Drawings

Fig. 1 illustrates an embodiment of the medical consultation support tool 100 according to the present invention;
Fig. 2 illustrates a screenshot 200 generated by the pre-consultation patient portal 102 in the embodiment 100 of Fig. 1 to obtain a patient's personal health information;
Fig. 3 illustrates a screenshot 300 generated by the pre-consultation patient portal 102 in the embodiment 100 of Fig. 1 to schedule an appointment;
Fig. 4 illustrates a screenshot 400 generated by the pre-consultation patient portal 102 in the embodiment 100 of Fig. 1 to obtain a patient's initial complaint description;
Fig. 5 illustrates a screenshot 500 generated by the pre-consultation patient portal 102 in the embodiment 100 of Fig. 1 to obtain symptom information as part of a patient's initial complaint description;
Fig. 6 illustrates a screenshot 600 generated by the pre-consultation patient portal 102 in the embodiment 100 of Fig. 1 to obtain additional symptom information based on anamnestic questions, automatically generated;
Fig. 7 illustrates a screenshot 700 generated by the clinician portal 104 in the embodiment of Fig. 1 to present the schedule of appointments to the clinician;
Fig. 8 illustrates a screenshot 800 generated by the clinician portal 104 in the embodiment of Fig. 1 to present the pre-consultation information obtained from the patient and the differential diagnose to the clinician and to obtain post consultation diagnosis information from the clinician; and
Fig. 9 shows a computing system suitable for hosting the medical consultation support tool according to the present invention, and suitable for implementing the method medical consultation support according to the present invention.

### Detailed Description of Embodiment(s)

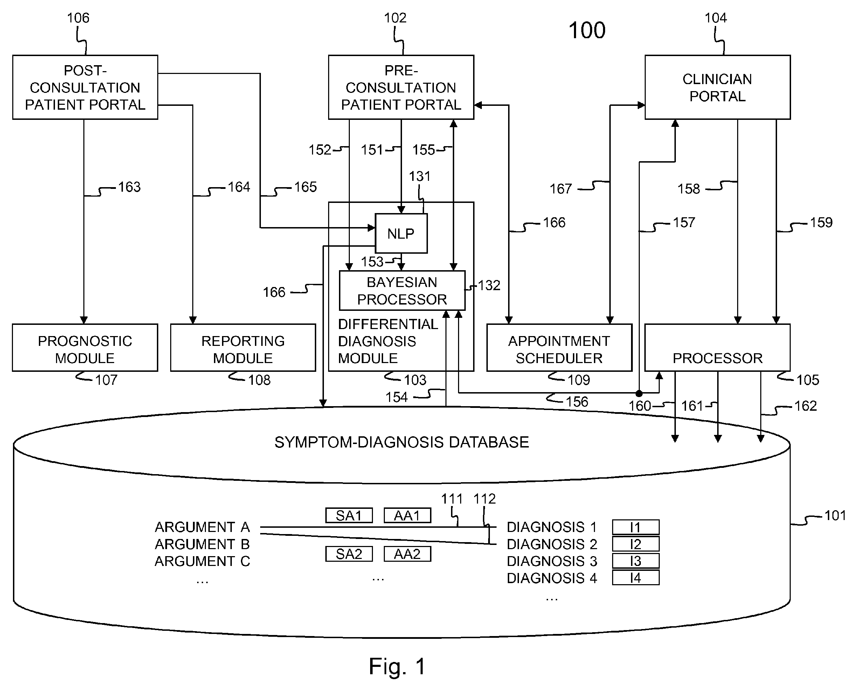

Fig. 1 shows an embodiment 100 of the medical consultation support tool according to the present invention. The medical consultation support tool 100 comprises a symptom-diagnosis database 101, a pre-consultation patient portal 102, a differential diagnosis module 103, a clinician portal 104, a processor 105, a post-consultation patient portal 106, a prognostic module 107, a reporting module 108 and an appointment scheduler 109. The differential diagnosis module 103 comprises a natural language processor or NLP 131 and a Bayesian statistics processor 132. The symptom-diagnosis database 101 maintains a list of arguments, e.g. ARGUMENT A, ARGUMENT B, ARGUMENT C, ..., a list of diagnoses, e.g. DIAGNOSIS 1, DIAGNOSIS 2, DIAGNOSIS 3, DIAGNOSIS 4, ..., incidence values I1, I2, I3, I4, ... for each of the diagnoses DIAGNOSIS 1, DIAGNOSIS 2, DIAGNOSIS 3, DIAGNOSIS 4, ..., sensitivity values SA1, SA2, ... linking arguments with diagnoses, and aspecificity values AA1, AA2, ... linking arguments with diagnoses. In the example of Fig. 1, the link 111 is shown between ARGUMENT A and DIAGNOSIS 1. For this link, two values are maintained, a sensitivity value denoted SA1 and an aspecificity value denoted AA1. Further, the link 112 is shown between ARGUMENT A and DIAGNOSIS 2. Also for this link, two values are maintained, a sensitivity value denoted SA2 and an aspecificity value denoted AA2. In a similar way, all arguments are linked to all diagnoses through respective sensitivity values and aspecificity values. These links are not shown in Fig. 1 to avoid overloading the drawing.

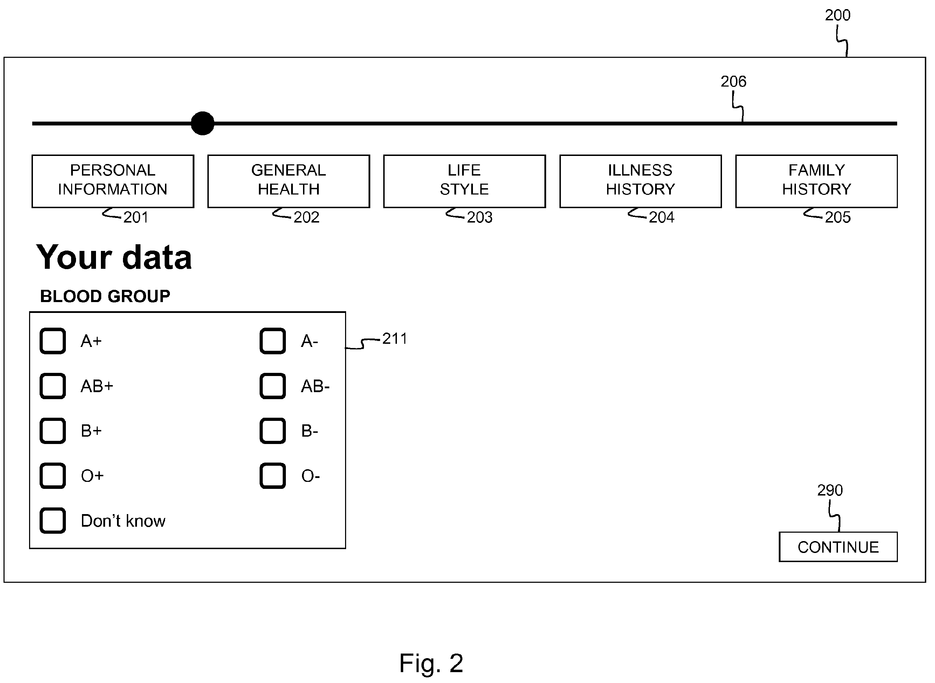

The pre-consultation patient portal 102 is a software module running with the patient on a client computer or running on a remote server, e.g. in a cloud computing system, and made accessible to the patient through a connected client device such as a personal computer, a laptop, a tablet computer, a smartphone, etc. The pre-consultation patient portal 102 is configured to enable the patient to enter and maintain his/her personal health record. Personal health information is obtained by the medical consultation support tool 100 through interrogation of the patient. This is illustrated by Fig. 2 which shows and exemplary screenshot 200 generated by the pre-consultation patient portal 102 to collect personal health information that forms part of the patient's personal health record. Fig. 2 illustrates that the tool 100 first collects personal information 201 such as name, contact information, blood group, person(s) to contact in case of emergency, etc. In Fig. 2, the patient can for instance enter his blood group 211 through clicking radio buttons displayed for all possible blood groups. By clicking the continue button 290, the patient shall sequentially be requested to enter general health information 202 such as information on allergies, medication, vaccinations, skin type, etc., life style information 203 such as the use of tobacco, the use of alcohol, sports frequency, sexual activeness, etc., illness history information 204 such as infection history, etc., and family history information 205 such as the occurrence of certain contagious illnesses or handicaps with relatives. The patient is informed on the progress made in entering his personal health information through the progress bar 206 displayed by the pre-consultation patient portal 102. The patient's personal health information is stored in a personal health record that can be retrieved and updated by the patient at any time.

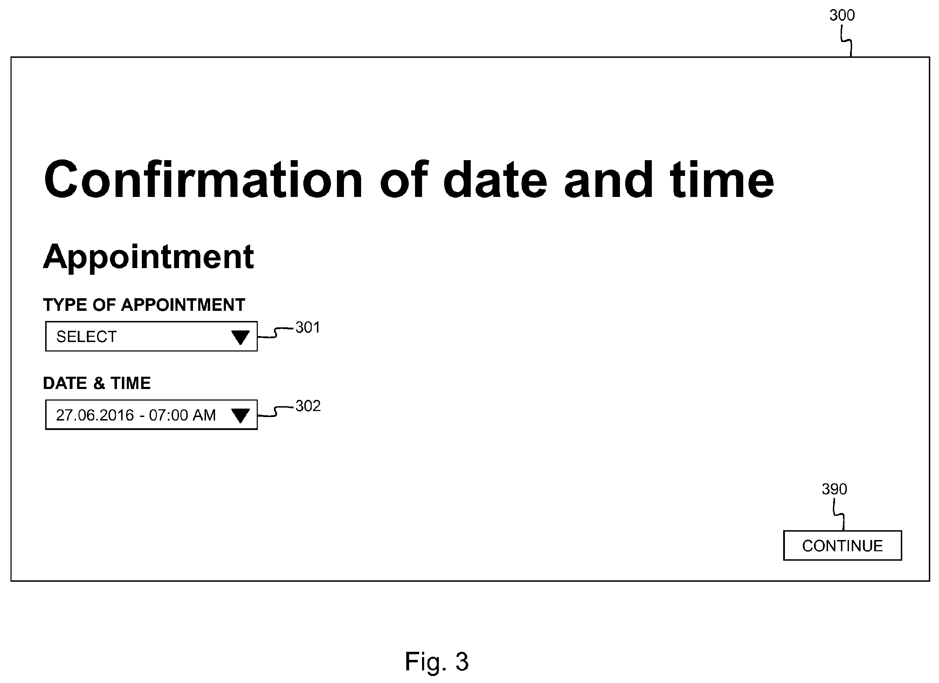

When the patient has a complaint, he/she can schedule an appointment with a clinician through the appointment scheduler 109 that forms part of the medical consultation support tool 100. Thereto, the pre-consultation patient portal 102 enables the patient to exchange appointment data 166 with the appointment scheduler 109. This is illustrated by Fig. 3 which shows an exemplary screenshot 300 generated by the pre-consultation patient portal 102 to enable the patient to select a preferred date and time 302 for his/her appointment and to indicate the type of appointment 301. The type of appointment 301 can for instance be selected from a number of options in a dropbox, e.g. a new complaint, a vaccination, delivery of a urine sample or blood sample, prescription for medication like anticonception, etc. The appointment scheduler 109 contains advanced features like automated adaptation of the length of the timeslot that can be reserved in field 302 as a function the selected type of appointment in field 301. The patient confirms his/her appointment via the continue button 390. The appointment scheduler 109 thereupon enters the appointment in the agenda of the clinician. This is indicated through arrow 167 in Fig. 1 where it is assumed that the clinician or his administrative staff consults his agenda via clinician portal 104, a software module that forms part of the consultation support tool 100 and either runs on a computer of the clinician or runs on a remote server, e.g. a cloud computing system, that is made accessible to the clinician via a client device such as a personal computer, laptop, tablet computer, smartphone or the like. The appointment scheduler 109 monitors annulations that result in free timeslots near the patient's appointment and automatically pushes information 166 on the occurrence of such free timeslots to the pre-consultation patient portal 102 to enable the patient to shift his/her appointment to a more preferred timeslot, e.g. an earlier timeslot or a timeslot at a more convenient time of the day. On the day of appointment, the appointment scheduler 109 monitors delays in the clinician's scheme and sends real-time updates 166 to the pre-consultation patient portal to enable the patient to arrive later or earlier and use the delay time in the clinician's scheme for instance for shopping or administration.

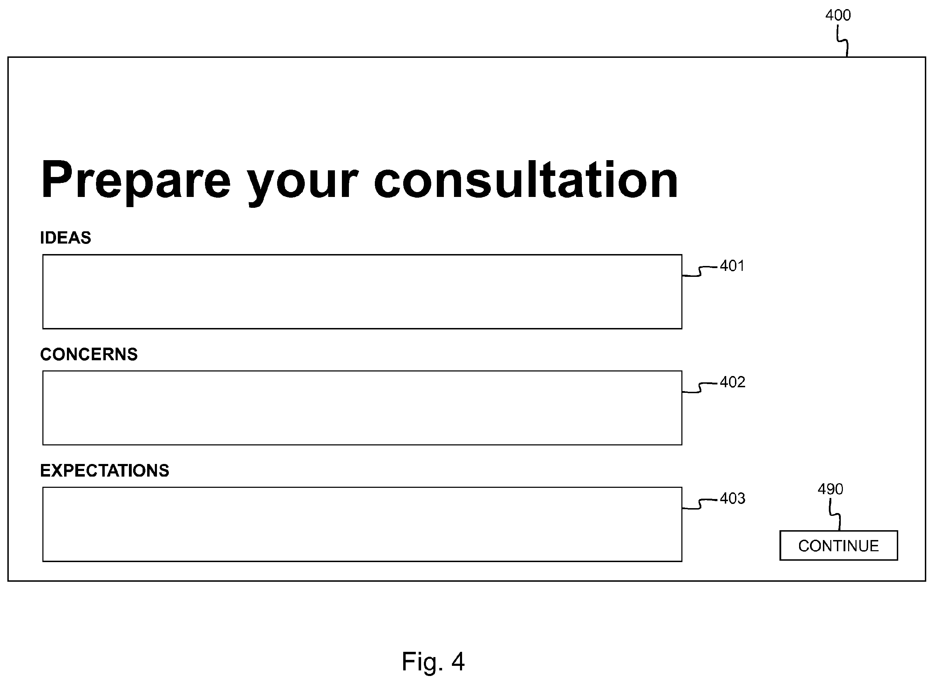

The pre-consultation patient portal 102 further is configured to enable the patient to prepare his/her scheduled consultation with the clinician. The consultation support tool 100 obtains the patient's thinking 151 and thereafter an initial complaint description 152 through interrogation of the patient. The patient's thinking 151 is obtained for instance through Ideas-Concerns-Expectations or ICE interrogation as illustrated by Fig. 4. The screenshot 400 shown there illustrates that the patient is requested to enter his ideas in a first free text field 401, his concerns in a second free text field 402, and his expectations in a third free text field 403. The patient confirms by clicking the continue button 490 as a result of which the free text information is transferred to the system. The information 151 obtained through ICE interrogation of the patient will later be shared with the clinician and may help the clinician to guide the consultation. This information 151 may be processed through the Natural Language Processor 131 for arguments and/or quantification of arguments.

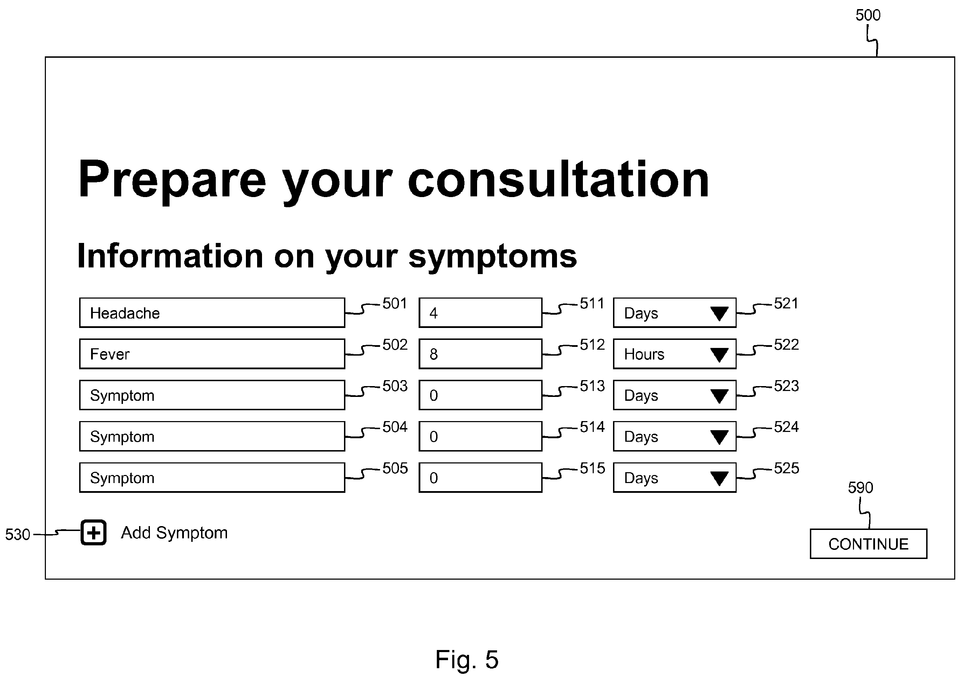

In addition to the ICE-based free text interrogation, the pre-consultation patient portal 102 is configured to interrogate the patient with respect to his symptoms or complaints. This is illustrated by Fig. 5 which shows a screenshot 500 presented to the patient to enter symptoms, e.g. "Headache" in field 501, "Fever" in field 502, and further possible symptoms in fields 503, 504 and 505. The symptoms entered can be selected from a dropbox or list of symptoms, free text can be entered, or even speech may be allowed in certain embodiments of the invention. In addition to identifying the symptoms, the patient has to indicate how long the respective symptoms 501-505 are present via respective fields 511-515 wherein the duration is quantified and fields 521-525 that operate as dropboxes and enable the patient to select the time unit to quantify the duration. Should the patient desire to enter additional symptoms, the button 530 enables to create additional lines similar to 501-511-521 for additional symptoms. Again, the patient confirms via the continue button 590 as a result of which the symptom information will be shared between the pre-consultation patient portal 102 and the Bayesian processor 132 that forms part of the differential diagnosis module 103. In Fig. 1, this is indicated by arrow 152.

Optionally, a natural language processor 131 may translate the patient complaint or free text / speech portions thereof into correct medical terms or arguments that can be used by the Bayesian engine 132 and reports the extracted symptoms or arguments 153 to the Bayesian processor 132.

The differential diagnosis module 103, more particularly the Bayesian statistics processor 132 therein, consults the symptom-diagnosis database 101 as is indicated by arrow 154, and matches the symptoms or arguments extracted from the personal health record and the patient's symptom/complaint description 152 with the arguments present in the database 101. Thereto a Bayesian statistic algorithm is applied and as a result thereof a differential diagnosis 156 is generated that contains a shortlist of for instance 10 to 20 potential diagnoses.

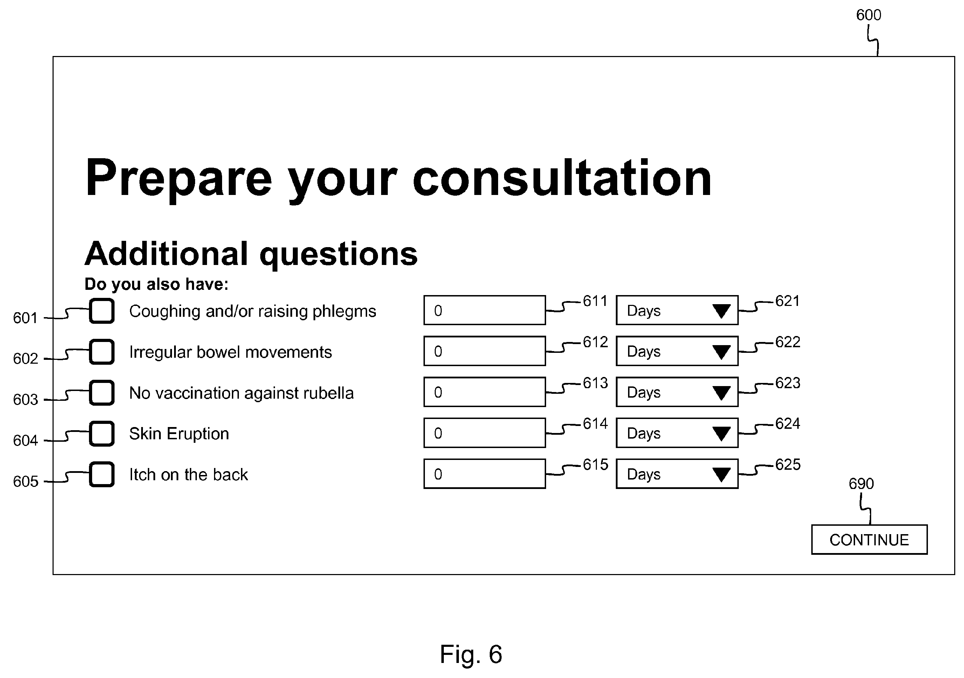

In a next step, the differential diagnosis module 103 generates anamnestic questions, i.e. a set of additional questions on symptoms that are strongly correlated with potential diagnoses in the differential diagnose in order to enable to rule out certain potential diagnoses or to intensify the probability of certain potential diagnoses. Starting from the potential diagnoses that form part of the differential diagnose, the Bayesian processor 132 selects arguments that have the highest sensitivity value correlating them with the potential diagnoses or that have the highest aspecificity value correlating them with the potential diagnoses. The so selected arguments are then presented to the patient. For arguments/symptoms with the highest sensitivity values, the patient is requested to confirm presence or absence and eventually to quantify these arguments. Also for arguments/symptoms with the highest aspecificity values, the patient is requested to confirm presence or absence. The additional questions "Do you also have ..." hence are generated automatically from the potential diagnoses in the differential diagnose and transferred to the pre-consultation patient portal as is indicated by arrow 155. Fig. 6 shows a screenshot 600 wherein these additional questions 601, 602, 603, 604, 605 are raised and the patient is required to quantify the presence or absence of the additional symptoms through respective fields 611-615 and 621-625. After having entered replies to the additional questions 601-605, the patient confirms by clicking the continue button 690.

Possibly, plural rounds of additional questions are generated and raised to the patient. The differential diagnosis module 103, upon receipt of the answers to the additional questions 155 adapts the differential diagnosis. This differential diagnosis 156 or 157, i.e. a list of for instance 10 potential diagnoses and their respective probabilities, is transferred to the clinician portal 104 and to processor 105.

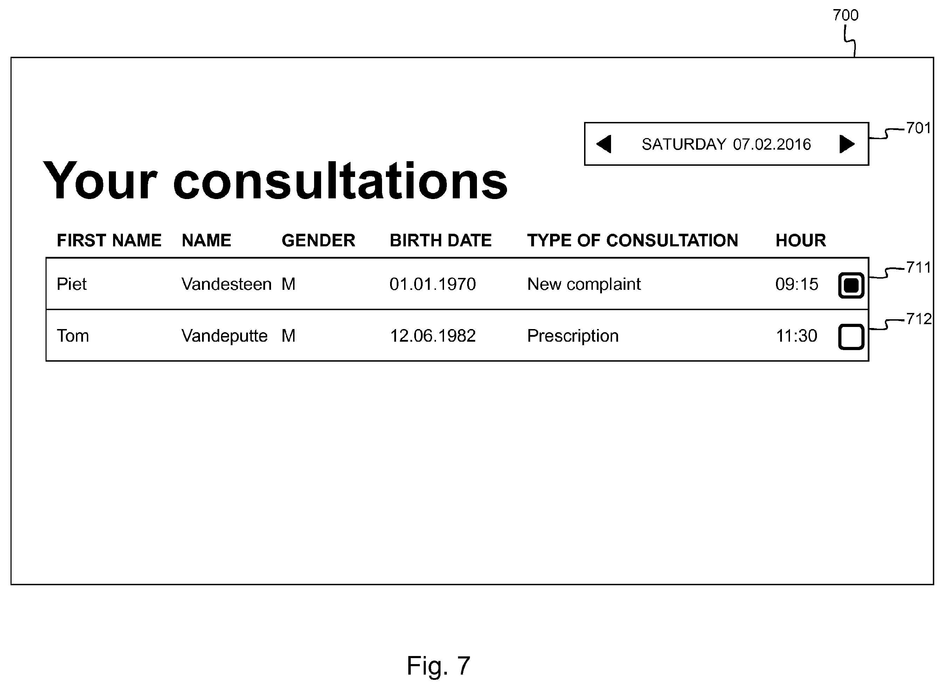

Fig. 7 shows a screenshot 700 generated by the clinician portal 104 to give the clinician an overview on his agenda. The clinician selects the period in field 701, for instance Saturday 7 February 2016. As a result, the appointments 711, 712 scheduled for that day are listed. The clinician for instance has a first appointment 711 concerning a new complaint with a male patient named Piet Vandesteen, born on 1 January 1970. This appointment is scheduled for 09:15 AM. The clinician further has a second appointment 712 with a male patient named Tom Vandeputte, born on 12 June 1982. This patient needs a prescription for medication and his appointment is scheduled at 11:30 AM.

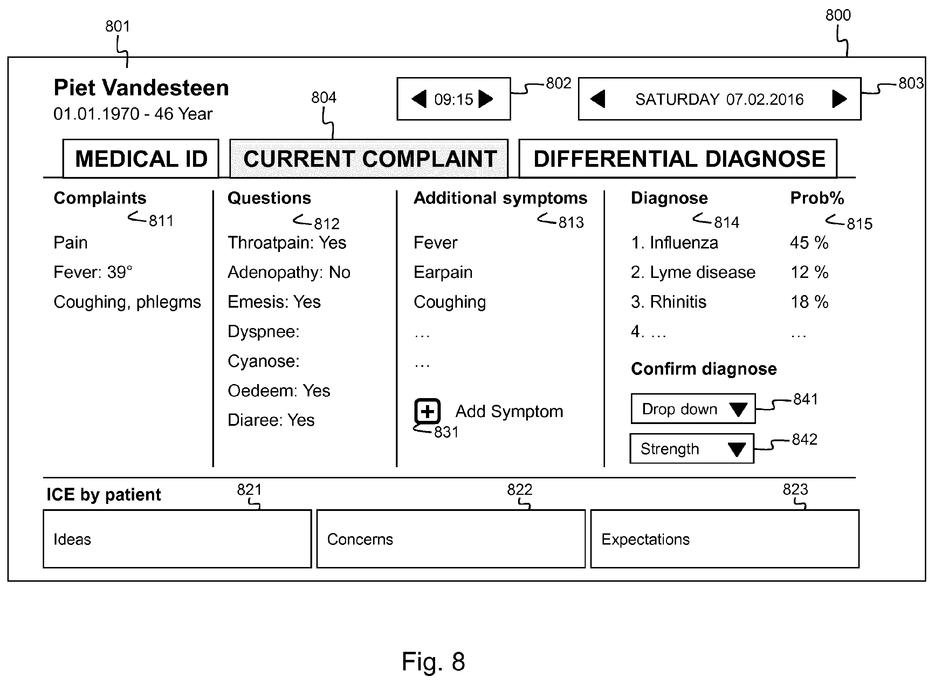

When clicking on the button near one of the appointments, e.g. appointment 711, the clinician portal 104 generates a screen 800 as depicted in Fig. 8 containing the pre-consultation information received from the patient and the differential diagnose generated by the differential diagnosis module 103. Screen 800 contains a first field 801 wherein the patient's personal details and type of consultation are displayed, and second and third fields 802, 803 respectively displaying the time and date of the appointment. In the current complaint tab 804, screen 800 further contains a field 811 wherein the complaints extracted from the patients initial complaint description 152 and their quantification as well as the additional questions are listed, fields 821, 822 and 823 wherein the free text information obtained from the patient in response to the ICE (Ideas, Concerns, Expectations) interrogation is displayed, a field 812 presenting the answers to the anamnestic questions raised by the Bayesian processor in the earlier digital anamnesis, a field 813 wherein the clinician enters additional symptoms seen during the consultation with button 831 to extend this field 813 with further symptoms, a field 814 wherein the differential diagnose is displayed and a field 815 wherein the respective probabilities of the potential diagnoses that form part of the differential diagnose 814 are listed. The clinician can confirm his diagnose via a drop down box 841 and his confidence 842 in the diagnose 841.

The final diagnosis 158 is transferred to the processor 105. Possibly, the clinician also may enter new symptoms 813 or 159 that result from tests or measurements during the consultation. As a result of such new symptoms 159, the differential diagnosis module 103 may adapt the differential diagnose 814, 815 or 156, 157, and the clinician may adapt his final diagnose 841 or 158.

Upon receipt of the final diagnosis and confidence information from the clinician, the processor 105 adapts the incidence values I1, I2, I3, I4, ..., the sensitivity values SA1, SA2, ... and the aspecificity values AA1, AA2, ... in the symptom-diagnosis database 101. The incidence value of the final diagnose of the clinician is increased with a value that is calculated from the confidence the clinician has in his final diagnose. The incidence increase is a rising function of this confidence value: the higher the confidence in the final diagnose, the higher the increase of the incidence value of this final diagnose in the database. Further, the sensitivity values are increased between the final diagnosis of the clinician and all symptoms/arguments that form part of the patient's complaint description, or additional symptoms/arguments entered by the clinician as a result of the consultation or presented by the differential diagnosis module to the patient as a result of the anamnestic additional question generation. Again, the increase in sensitivity between such symptoms/arguments and the final diagnosis is calculated from the confidence the clinician has in his diagnosis. The increase in sensitivity is also a rising function of the confidence value: the higher the confidence in the final diagnose, the higher the sensitivity between identified symptoms and final diagnose is increased. Further, also the aspecificity values are increased between the identified symptoms, either through the patient's complaint description, the additional questions or the clinician consultation, and all diagnoses that form part of the differential diagnosis but differ from the clinician's final diagnosis. The increase in aspecificity values for such argument-diagnosis tuples is again calculated from the confidence value in the final diagnosis, and also is a rising function thereof. The increase values for incidence values, sensitivity values and aspecificity values are communicated by the processor 105 to the database 101 as is indicated by arrow 162. The database 101 stores the adapted values.

The system 100 drawn in Fig. 1 further comprises a post-consultation patient portal 106, i.e. a software module that is either installed and running on a client computer with the patient, or that is installed and running remotely on a server, e.g. a cloud computing server, and made remotely accessible to a client device, e.g. a personal computer, a laptop, a tablet computer, a smartphone or the like. The post-consultation patient portal 106 enables to interrogate the patient in the days, weeks or months following his consultation with the clinician. The patient is interrogated with respect to the effect of the treatment and possibly the medication prescribed by the clinician. The treatment effectiveness information 163 obtained from the patient is used by a prognostic module 107 that generates prognoses for later patients with for instance similar diagnose, gender, age, comorbidities, concomitant medication and prescribed medication. The skilled person will appreciate that the just mentioned list is non-exhaustive. The prognostic module 107 for instance calculates the average recovery time of earlier patients with same diagnose, and prescribed medication, with same gender and whose age lies within a certain age interval around the age of a new patient.

The post-consultation patient portal 106 further interrogates the patient with respect to possible side-effects of the prescribed medication. The side-effect feedback 164 received from the patients on large scale is statistically processed by a reporting module 108 which further generates reports for the pharma industry on the side-effects of their medication.

Further, the post-consultation patient portal 106 requests the patient to reformulate his complaint at a point in time he/she is more literate on the medical problem. The reformulated complaint description 165 is submitted to the natural language processor NLP 131 and the NLP 131 may compare the reformulated complaint description with initial free text or speech received from the patient in order to identify alternate symptom descriptions therein. A comparison between the reformulated complaint description 165 and the initial free text or speech complaint description enables the natural language processor 131 to identify alternate wording or phrasing for symptoms/arguments in the database 101. These alternate wording or phrasing may for instance comprise dialect expressions used by patients. The natural language processor 131 informs the database 101 on such alternate terminology 166 and the database 101 is enriched through storage of such alternate terminology. In future free text or speech complaint descriptions of patients, the dialect or alternate expressions used to describe certain symptoms will be recognised as a result of which the differential diagnosis will become more accurate.

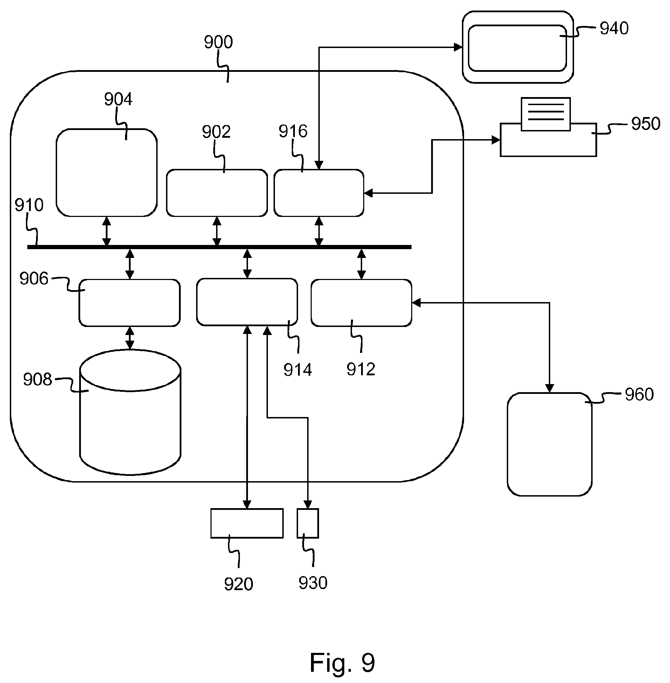

Fig. 9 shows a suitable computing system 900 for hosting the medical consultation support tool according to the invention, an embodiment of which is drawn in Fig. 1. Computing system 900 may in general be formed as a suitable general purpose computer and comprise a bus 910, a processor 902, a local memory 904, one or more optional input interfaces 914, one or more optional output interfaces 916, a communication interface 912, a storage element interface 906 and one or more storage elements 908. Bus 910 may comprise one or more conductors that permit communication among the components of the computing system. Processor 902 may include any type of conventional processor or microprocessor that interprets and executes programming instructions. Local memory 904 may include a random access memory (RAM) or another type of dynamic storage device that stores information and instructions for execution by processor 902 and/or a read only memory (ROM) or another type of static storage device that stores static information and instructions for use by processor 904. Input interface 914 may comprise one or more conventional mechanisms that permit an operator to input information to the computing device 900, such as a keyboard 920, a mouse 930, a pen, voice recognition and/or biometric mechanisms, etc. Output interface 916 may comprise one or more conventional mechanisms that output information to the operator, such as a display 940, a printer 950, a speaker, etc. Communication interface 912 may comprise any transceiver-like mechanism such as for example two 1 Gb Ethernet interfaces that enables computing system 900 to communicate with other devices and/or systems, for example mechanisms for communicating with one or more other computing systems. The communication interface 912 of computing system 900 may be connected to such another computing system 960 by means of a local area network (LAN) or a wide area network (WAN), such as for example the internet, in which case the other computing system 960 may for example comprise a suitable web server. Storage element interface 906 may comprise a storage interface such as for example a Serial Advanced Technology Attachment (SATA) interface or a Small Computer System Interface (SCSI) for connecting bus 910 to one or more storage elements 908, such as one or more local disks, for example 1TB SATA disk drives, and control the reading and writing of data to and/or from these storage elements 908. Although the storage element 908 above is described as a local disk, in general any other suitable computer-readable media such as a removable magnetic disk, optical storage media such as a CD or DVD, -ROM disk, solid state drives, flash memory cards, ... could be used.

The steps executed in the method for medical consultation support according to the present invention, illustrated by the above embodiments, may be implemented as programming instructions stored in local memory 904 of the computing system 900 for execution by its processor 902. Alternatively the instructions may be stored on the storage element 908 or be accessible from another computing system 960 through the communication interface 912.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being defined by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. It is to be understood that the terms so used are interchangeable under appropriate circumstances and embodiments of the invention are capable of operating according to the present invention in other sequences, or in orientations different from the one(s) described or illustrated above.

## Claims

1. A medical consultation support tool (100) comprising:
- a database (101) of arguments and diagnoses wherein an incidence value (I1, I2, I3, I4) is maintained for each diagnosis, i.e. a value indicating the occurrence probability of the disease corresponding to said diagnosis, and wherein an argument and diagnosis are relationally coupled through a sensitivity value (SA1, SA2), i.e. a value indicating how often said argument is present in case a patient has the disease corresponding to said diagnosis, and through an aspecificity value (AA1, AA2), i.e. a value indicating how often said argument is present in case a patient does not have the disease corresponding to said diagnosis;
- an electronic patient portal (102) adapted to obtain a pre-consultation complaint description (152) from a patient; and
- a differential diagnosis module (103) adapted to generate a differential diagnosis (156) through matching arguments identified in said pre-consultation complaint description (152) with arguments present in said database (101) and determining potential diagnoses from incidence values, sensitivity values and aspecificity values, wherein said differential diagnosis (156) comprises a list of potential diagnoses (701-710) and respective probabilities (711-720) of said potential diagnoses (701-710),
**CHARACTERIZED in that** said medical diagnosis support tool (100) further comprises:
- an electronic clinician portal (104) adapted to obtain post-consultation diagnosis information and confidence information (158) from a clinician; and
- a processor (105) configured to automatically adapt incidence values (I1, I2, I3, I4) in said database (101) for diagnoses in said post-consultation diagnosis information (158), and sensitivity values (SA1, SA2) and aspecificity values (AA1, AA2) in said database (101) for arguments identified in said pre-consultation complaint description (152) with respective amounts calculated from said post-consultation diagnosis information and said confidence information (158) obtained from said clinician.

2. A medical consultation support tool (100) according to claim 1, wherein:
- said differential diagnosis module (103) and said electronic patient portal (102) are further adapted to present to said patient one or more additional arguments (601-605), to obtain confirmation or denial for said additional arguments (601-605) from said patient, and to generate an update of said differential diagnosis (156) through matching said arguments identified in said pre-consultation complaint description (152) and said additional arguments confirmed or denied by said patient with arguments present in said database; and
- said processor (105) is further configured to automatically adapt sensitivity values and aspecificity values in said database (101) for said additional arguments with respective amounts calculated from said post-consultation diagnosis information and said confidence information obtained from said clinician.

3. A medical consultation support tool (100) according to claim 1 or claim 2, wherein:
- said electronic clinician portal (102) is further adapted to enable said clinician to add one or more additional arguments;
- said differential diagnosis module (103) is further adapted to re-generate a differential diagnosis through matching said arguments identified in said pre-consultation complaint description (152) and said additional arguments (155) with arguments present in said database (101) and determining potential diagnoses from incidence values, sensitivity values and aspecificity values; and
- said processor (105) is further configured to automatically adapt sensitivity values and aspecificity values in said database (101) for said additional arguments with respective amounts calculated from said post-consultation diagnosis information and said confidence information (158) obtained from said clinician.

4. A medical consultation support tool (100) according to claim 1 or claim 2 or claim 3,
wherein said processor (105) is configured to increase a sensitivity value for arguments identified in said pre-consultation complaint description (152) or additional arguments (155, 159) confirmed by said patient or added by said clinician and a diagnosis corresponding with said diagnosis information (158) of said clinician, the increase amount of said sensitivity value being determined by said confidence information (158) of said clinician.

5. A medical consultation support tool (100) according to claim 1 or claim 2 or claim 3,
wherein said processor (105) is configured to increase an aspecificity value for arguments identified in said pre-consultation complaint description (152) or additional arguments (155, 159) confirmed by said patient or added by said clinician and a potential diagnosis that forms part of said differential diagnosis (156) but differs from said diagnosis information (158) of said clinician, the increase amount of said aspecificity value being determined by said confidence information (158) of said clinician.

6. A medical consultation support tool (100) according to claim 1 or claim 2 or claim 3,
wherein said processor (105) is configured to increase an incidence value for a diagnosis that corresponds with said diagnosis information (158) of said clinician, the increase amount of said incidence value being determined by said confidence information (158) of said clinician.

7. A medical consultation support tool (100) according to one of the preceding claims, wherein:
- said processor (105) is further configured to add a new diagnosis to said database in case a diagnosis corresponding with said diagnosis information of said clinician is not present in said database; and
- said processor is further configured to create sensitivity values and aspecificity values in said database for arguments identified in said pre-consultation complaint description or additional arguments and said new diagnosis.

8. A medical consultation support tool (100) according to one of the preceding claims, further comprising:
- a natural language processor (131), coupled to said patient portal (102) and adapted to process free text or speech portions of said pre-consultation complaint description (152) to identify one or more arguments therein.

9. A medical consultation support tool (100) according to claim 8,
wherein said natural language processor (131) is further adapted to quantify certain arguments identified from said free text or speech portions of said pre-consultation complaint description.

10. A medical consultation support tool (100) according to one of the preceding claims, further comprising:
- an electronic patient portal (106) for post-consultation interrogation of said patient, adapted to collect treatment effectiveness information (163) and/or medication effectiveness information (164) from said patient.

11. A medical consultation support tool (100) according to claim 10, further comprising:
- a prognostic module (107) coupled to said electronic patient portal (106) for post-consultation interrogation and adapted to calculate an estimated intention time for a new patient from treatment effectiveness information (163) obtained from earlier patients with same diagnosis and prescribed medication as said new patient.

12. A medical consultation support tool (100) according to claim 10, further comprising:
- a reporting module (108) coupled to said electronic patient portal (106) for post-consultation interrogation and adapted to generate statistical reports on side effects of medication from said medication effectiveness information (164) obtained from patients.

13. A medical consultation support tool (100) according to one of the preceding claims, further comprising:
- an electronic appointment scheduler (109) enabling said patient to schedule an appointment with a clinician, to automatically receive real-time updates on said appointment within a certain time interval preceding said appointment, and to automatically receive reschedule suggestions upon appointment cancellations by other patients.

14. A medical consultation support tool (100) according to one of the preceding claims, further comprising:
- an electronic patient portal (106) for post-consultation interrogation of said patient, adapted to collect a reformulated complaint description (165) from said patient; and
- a processor (103) coupled to said electronic patient portal (106) for post-consultation interrogation and adapted to enrich said database (101) of arguments and diagnoses with alternate argument descriptions extracted from a comparison between said pre-consultation complaint description (152) and said reformulated complaint description (165).

15. A computer-implemented method for medical consultation support, comprising the steps of:
- maintaining a database (101) of arguments and diagnoses wherein an incidence value (I1, I2, I3, I4) is maintained for each diagnosis, i.e. a value indicating the occurrence probability of the disease corresponding to said diagnosis, and wherein an argument and diagnosis are relationally coupled through a sensitivity value (SA1, SA2), i.e. a value indicating how often said argument is present in case a patient has the disease corresponding to said diagnosis, and through an aspecificity value (AA1, AA2), i.e. a value indicating how often said argument is present in case a patient does not have the disease corresponding to said diagnosis;
- obtaining via an electronic patient portal (102) a pre-consultation complaint description (152) from a patient; and
- generating a differential diagnosis (156) through matching arguments identified in said pre-consultation complaint description (152) with arguments (154) present in said database (101) and determining potential diagnoses from incidence values, sensitivity values and aspecificity values, wherein said differential diagnosis (156) comprises a list of potential diagnoses (701-710) and respective probabilities (711-720) of said potential diagnoses,
**CHARACTERIZED in that** said method further comprises:
- obtaining post-consultation diagnosis information and confidence information (158) from a clinician via an electronic clinician portal (104); and
- automatically adapting incidence values (I1, I2, I3, I4) in said database (101) for diagnoses in said post-consultation diagnosis information (158), and sensitivity values (SA1, SA2) and aspecificity values (AA1, AA2) in said database (101) for arguments identified in said pre-consultation complaint description (152) with respective amounts calculated from said post-consultation diagnosis information and said confidence information (158) obtained from said clinician.
